# EUROPÄISCHE PATENTSCHRIFT

(11) **EP 1 515 954 B1**
(45) Veröffentlichungstag und Bekanntmachung des Hinweises auf die Patenterteilung: **24.02.2010**
(21) Anmeldenummer: 03735523.7
(22) Anmeldetag: 03.06.2003
(51) Int. Cl.: C07D 239/00

(54) **VERFAHREN ZUR HERSTELLUNG VON 4,6-DICHLOR-5-FLUORPYRIMIDIN**
METHOD FOR PRODUCING 4,6-DICHLORO-5-FLUOROPYRIMIDINE
PROCEDE DE FABRICATION DE 4,6-DICHLORO-5-FLUOROPYRIMIDINE

(30) Priorität: 13.06.2002 DE 10226220
(43) Veröffentlichungstag der Anmeldung: 23.03.2005
(73) Patentinhaber: Bayer CropScience AG, 40789 Monheim (DE)
(72) Erfinder: GELLER, Thomas, 51519 Odenthal (DE); WEINTRITT, Holger, 40764 Langenfeld (DE)
(86) Internationale Anmeldenummer: PCT/EP2003/005778
(87) Internationale Veröffentlichungsnummer: WO 2003/106432

(56) Entgegenhaltungen:
- EP-A- 1 077 210
- WO-A-95/29166

## Beschreibung

Die Erfindung betrifft ein neues Verfahren zur Herstellung von 4,6-Dichlor-5-fluorpyrimidin.

4,6-Dichlor-5-fluor-pyrimidin ist ein Zwischenprodukt, das beispielsweise für die Herstellung von Pflanzenschutzmitteln (vgl. EP-A-0 882 043) eingesetzt werden kann.

Ein Verfahren zur Herstellung von 4,6-Dichlor-5-fluor-pyrimidin ist bereits in EP-A-1 077 210 beschrieben.

In diesem Verfahren wird 4,6-Dihydroxy-5-fluor-pyrimidin oder dessen Alkalisalz mit Phosphoroxychlorid umgesetzt. Das dabei entstehende Reaktionsgemisch 1 wird mit Chlor in Gegenwart von Phosphortrichlorid umgesetzt. Aus dem dabei entstehenden Reaktionsgemisch wird das Produkt 4,6-Dihydroxy-5-fluor-pyrimidin von Phosphoroxychlorid abgetrennt. Nachteilig bei diesem Verfahren ist die Zurückführung von Phosphoroxychlorid durch eine aufwendige Destillation sowie die lange Reaktionsdauer.

Aus dem Stand der Technik sind Verfahren zur Chlorierung von Hydroxypyrimidinen bekannt. In diesen Verfahren werden unsubstituierte oder substituierte Hydroxypyrimidine mit Chlor und Phosphortrichlorid oder Phosphoroxychlorid zu den entsprechenden Chlor-substituierten Pyrimidinen umgesetzt (vgl. z.B. DE-A1-196 42533, DE-A1-195 242 83).

Die Verwendung von Phosgen als Chlorierungsreagenz zur Herstellung von Chlor-substituierten Pyrimidinen aus Hydroxy-substituierten Pyrimidinen ist dagegen außergewöhnlich.

Ein Verfahren zur Herstellung von 4,6-Dichlorpyrimidin, das in 5-Stellung nicht substituiert ist, wird in WO 95/29166 beschrieben. Bei diesem Verfahren wird 4,6-Dihydroxypyrimidin mit Phosgen in Gegenwart einer Base und gegebenenfalls in Gegenwart eines Lösungsmittels umgesetzt.

Ein weiteres Verfahren zur Herstellung von unsubstituiertem 4,6-Dichlorpyrimidin wird in WO 02/00628 beschrieben. In diesem Verfahren wird 4,6-Dihydroxypyrimidin mit Phosgen in Gegenwart eines quartären Ammoniumsalzes oder quartären Phosphoniumsalzes als Katalysator mit Phosgen umgesetzt.

Aus WO 00/46212 ist ein Verfahren zur Herstellung von in 2-Stellung substituiertem 4-Chlorpyrimidin bekannt. Bei diesem Verfahren wird ein in 2-Stellung substituiertes 4-Hydroxypyrimidin mit Phosgen in Gegenwart eines Phosphans oder Phosphanoxids als Katalysator und gegebenenfalls eines Phasentransferkatalysators umgesetzt.

In diesen Verfahren werden als Ausgangsverbindungen entweder unsubstituierte oder in 2-Stellung substituierte Hydroxypyrimidine verwendet.

Nur in FR-A-1310810 wird ein Verfahren offenbart, welches die Chlorierung von in 5-Stellung durch Aryl- oder Alkyl-substituierten Hydroxypyrimidinen mit Phosgen ermöglicht. Beispielhaft wird die Umsetzung von 5-Phenyl-4,6-dihydroxypyrimidin in o-Dichlorbenzol als Lösungsmittel und Dimethylformamid als Katalysator zu 5-Phenyl-4,6-dichlorypyrimidin beschrieben. Nachteilig bei diesem Verfahren ist, dass Phosgen mit Dimethylformamid ein krebserregendes Zwischenprodukt bildet.

Es ist kein Verfahren bekannt, das die Chlorierung von in 5-Stellung Halogensubstituierten Hydroxypyrimidinen mit Phosgen erlaubt.

Aufgabe der vorliegenden Erfindung war es nun, ein Verfahren zur Herstellung von 4,6-Dichlor-5-fluor-pyrimidin bereitzustellen, das die Darstellung in großtechnischem Maßstab in besseren Ausbeuten, höherer Reinheit und kürzeren Reaktionszeiten ermöglicht. Insbesondere sollte ein Verfahren gefunden werden, bei dem eine aufwendige Destillation und die Rückführung bzw. Entsorgung von Phosphoroxychlorid vermieden werden.

Es wurde nun überraschenderweise gefunden, dass man das 5-Fluor-substituierte 4,6-Dichlorpyrimidin der Formel 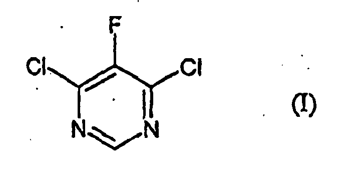 erhält, wenn man
4,6- Dihydroxy-5-fluor-pyrimidin der Formel (II) oder dessen Alkalisalz 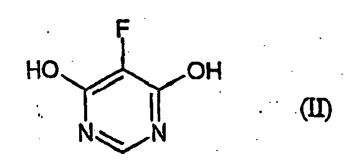 mit Phosgen in Gegenwart von Nitrobenzol als Lösungsmittel und in Gegenwart eines Katalysators umsetzt.

In einer Variante wird die Umsetzung in Gegenwart von Nitrobenzol als Lösungsmittel und in Gegenwart von 4-Dimethylaminopyridin (DMAP) als einzigem Katalysator durchgeführt.

Ein deutlicher Vorteil des erfindungsgemäßen Verfahrens liegt darin, dass 4,6-Dichlor-5-fluor-pyrimidin im Vergleich zu dem einzigen bekannten Herstellungsverfahren (vgl. EP-A-1 077 210) in wesentlich höheren Ausbeuten erhalten wird.

Ein weiterer Vorteil ist, dass der Anteil an Nebenprodukten sehr gering ist.

Bei der Produktisolierung ist es ein deutlicher Vorteil, dass bei der Durchführung des Verfahrens in Nitrobenzol als Lösungsmittel und 4-Dimethylaminopyridin (DMAP) als Katalysator das 4,6-Dichlor-5-fluor-pyrimidin als erste Komponente des Reaktionsgemisches abdestilliert werden kann und der Destillationssumpf aus Nitrobenzol, DMAP und gegebenenfalls vorhandenen Nebenkomponenten für eine weitere Umsetzung wiederverwendet werden kann.

4,6-Dihydroxy-5-fluor-pyrimidin der Formel (II) ist bekannt und kann durch einfache Verfahren hergestellt werden (vgl. EP-A-1 079 210).

Alle anderen Ausgangsverbindungen sind gängige Handelsprodukte oder können durch einfache Verfahren aus diesen hergestellt werden.

Phosgen kann gasförmig oder in gelöster Form verwendet werden.

Als Katalysatoren des erfindungsgemäßen Verfahrens kommen Triarylphosphanoxide wie beispielsweise Triphenylphosphanoxid; Trialkylphosphanoxide wie beispielsweise Tri-n-octylphosphanoxid, Tri-n-butylphosphanoxid; Triarylphosphane wie beispielsweise Triphenylphosphan; Trialkyl-phosphane wie beispielsweise Tri-n-octylphosphan; substituierte Harnstoffe wie beispielsweise Tetrabutylhamstoff, Tetramethylharnstoff; N,N-Dimethylformamid; oder Amine wie beispielsweise 4-Dimethylaminopyridin (DMAP) oder Piperidin in Betracht. Bevorzugt wird DMAP verwendet.

Die Reaktionstemperaturen können bei der Durchführung des erfindungsgemäßen Verfahrens in einem größeren Bereich variiert werden. Im Allgemeinen arbeitet man in einem Temperaturbereich von 40°C bis Rückflusstemperatur der jeweiligen Mischung, vorzugsweise in einem Temperaturbereich von 60 bis 120°C.

Das erfindungsgemäße Verfahren wird bei Normaldruck oder bei erhöhtem Druck durchgeführt, insbesondere bei Drücken von 1 bis 4 bar, bevorzugt bei Drücken von Normaldruck bis 3 bar.

Zur Durchführung des erfindungsgemäßen Verfahrens zur Herstellung der Verbindungen der Formel (I) setzt man pro Mol der Verbindung der Formel (II) im allgemeinen 2 bis 20 Mol, vorzugsweise 2 bis 10 Mol, besonders bevorzugt 2 bis 6 Mol Phosgen ein.

Zur Durchführung des erfindungsgemäßen Verfahrens zur Herstellung der Verbindung der Formel (I) setzt man pro Mol der Verbindung der Formel (II) im allgemeinen 0 bis 30 Mol-%, vorzugsweise 0 bis 10 Mol-%, besonders bevorzugt 0 bis 5 Mol-% des Katalysators ein.

Zur Durchführung des erfindungsgemäßen Verfahrens zur Herstellung der Verbindung der Formel (I) setzt man pro Mol der Verbindung der Formel (II) im allgemeinen 0-30 Mol-%, vorzugsweise 0-10 Mol-%, besonders bevorzugt 0-5 Mol-% des Phasentransferkatalysators ein.

Zur Durchführung des erfindungsgemäßen Verfahrens geht man im Allgemeinen wie folgt vor:
Die Verbindung der Formel (II) wird in einem Lösungsmittel, gegebenenfalls mit einem Katalysator vorgelegt. Es wird Phosgen eingeleitet oder gegebenenfalls in flüssiger Form zugesetzt und die Reaktionsmischung zwischen einer und 24 Stunden erhitzt. Anschließend erfolgt eine destillative Aufarbeitung.

Das erfindungsgemäße Verfahren wird zur Herstellung von 4,6-Dichlor-5-fluor-pyrimidin (I) verwendet, das ein wichtiges Zwischenprodukt für die Herstellung von Schädlingsbekämpfungsmitteln ist. Nach dem erfindungsgemäßen Verfahren kann man 4,6-Dichlor-5-fluor-pyrimidin mit konstant hohen Reinheiten und sehr guten Ausbeuten erhalten. Das neue Verfahren erleichtert daher die Herstellung von bekannten und neuen Schädlingsbekämpfungsmitteln.

Die nachfolgenden Beispiele dienen zur Erläuterung der Erfindung. Die Erfindung ist jedoch nicht auf die Beispiele limitiert.

### Beispiele

### Beispiele 1, 2, 8-12, 15-22 zum Vergleich; Beispiele 23-27 und 29-34 erfindungsgemäß

### Allgemeine Vorschrift 1 für die Herstellung von 4,6-Dichlor-5-fluorpyrimidin aus 4,6-Dihydroxy-5-fluorpyrimidin

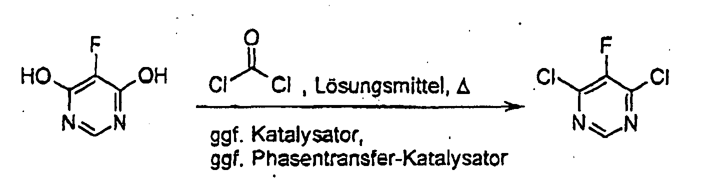

In einem Dreihalskolben mit Rührer, Innenthermometer sowie einem auf -30°C gekühlten Rückflusskühler mit aufgesetzter Gasableitungskappe und Anschluss an einen Waschturm werden 1.30 g (10 mmol) 4,6-Dihydroxy-5-fluorpyrimidin vorgelegt und gegebenenfalls (ggf.) mit Katalysator versetzt (Art und Äquivalente: siehe Tabelle). Nach Zugabe von 40 g einer 15 %igen Lösung von Phosgen in einem Lösungsmittel (= 6 eq. Phosgen, Lösungsmittel: siehe Tabelle) wird der Ansatz bei einer Badtemperatur von 95°C (Chlorbenzol, Nitrobenzol) bzw. 75°C (Acetonitril) erhitzt und für 20 Stunden unter Phosgenrückfluss gerührt. Die Ausbeute wird durch Auswaage und Gehaltsbestimmungen der Reaktionsmischung gegen Standard (Reinsubstanz, GC/MS silyliert) ermittelt.

**Tabelle 1:**

| Beispiel | Lösungsmittel | Katalysator 1 (eq) | Katalysator 2 (eq) | Ausbeute d.Th.[%] |
|---|---|---|---|---|
| 1 | Chlorbenzol | / | / | / |
| 2 | Chlorbenzol | Triphenylphosphanoxid (0,1) | / | 82 |
| 8 | Chlorbenzol | Triphenylphosphanoxid (0,1) | 4-Dimethylaminopyridin (0,1) | 72 |
| 9 | Chlorbenzol | / | 4-Dimethylaminopyridin (0,1) | 74 |
| 10 | Chlorbenzol | / | Tetrabutylharnstoff (0,1) | 84 |
| 11 | Chlorbenzol | Tributylphosphanoxid (0,1) | / | 86 |
| 12 | Chlorbenzol | Trioctylphosphanoxid (0,1) | / | 88 |
| 15 | Chlorbenzol | / | N,N-Dibutylformamid (0,1) | 62 |
| 16 | Chlorbenzol | Triphenylphosphanoxid (0,1) | Tetr-n-butylharnstoff (0,1) | 91 |
| 17 | Chlorbenzol | / | Harnstoff (0,1) | 43 |
| 18 | Chlorbenzol | / | Tetra-n-methylhanistoff (0,1) | 72 |
| 19 | Chlorbenzol | / | Tetra-n-butylhamstoff (0,1) | 75 |
| 20 | Chlorbenzol | / | 1,3-Dimethylimidazolin-2-on (0,1) | 52 |
| 21 | Chlorbenzol | / | N-Methylpyrrolidon (0,1) | 22 |
| 22 | Chlorbenzol | Tri-n-octylphosphanoxid (0,1) | Tetra-n-butylharnstoff (0,1) | 90 |
| 23 | Nitrobenzol | / | / | 66 |
| 24 | Nitrobenzol | / | 4-Dimethylaminopyridin (0,1) | 93 |
| 25 | Nitrobenzol | / | 4-Dimethylaminopyridin (0,05) | 94 |
| 26 | Nitrobenzol | / | 4-Dimethylaminopyridin (0,025) | 94 |
| 27 | Nitrobenzol | / | 4-Dimethylaminopyridin (0,01) | 81 |
| 29 | Nitrobenzol | Tri-n- . butylphosphanoxid (0,1) | / | 88 |
| 30 | Nitrobenzol | Tri-n octylphosphanoxid (0,1) | / | 90 |
| 31 | Nitrobenzol | Triphenylphosphanoxid (0,1) | / | 88 |
| 32 | Nitrobenzol | Tri-n-octylphosphanoxid (0,1) | Tetramethylharnstoff (0,1) | 84 |
| 33 | Nitrobenzol | / | Pyridin (0,1) | 79 |
| 34 | Nitrobenzol | / | Imidazol (0,1) | 83 |
| 36 | Acetonitril | Triphenylphospbanoxid (0,1) | 4-Dimethylaminopyridin (0,1) | 79 |
| 37 | Acetonitril | / | Tetra-n-butyihamstoff (0,1) | 55 |
| 38 | Acetonitril | / | Teramethylharnstoff (0,1) | 53 |

### Allgemeine Vorschrift 2 für die Herstellung von 4,6-Dichlor-5-fluorpyrimidin aus 4,6-Dihydroxy-5-fluorpyrimidin (für der Darstellung der Vergleichsbeispiele 39-40 und 44-46)

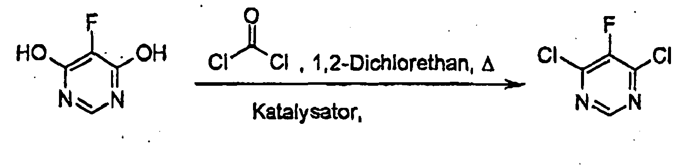

In einem Dreihalskolben mit Rührer, Innenthermometer sowie einem auf -30°C gekühlten Rückflusskühler mit aufgesetzter Gasableitungskappe und Anschluss an einen Waschturm werden 6.5 g (50 mmol) 4,6-Dihydroxy-5-fluorpyridin vorgelegt und mit Katalysator versetzt (Art und Äquivalente: siehe Tabelle). Nach Zugabe von 175 ml 1,2-Dichlorethan werden 4 eq. Phosgen bei ca. 70 - 80°C eingeleitet und für 6 Stunden unter Phosgenrückfluss und anschließend für ca. 14 Stunden ohne Phosgenrückfluss (Kühler abgeschaltet) gerührt. Die Ausbeute wird durch Auswaage und Gehaltsbestimmungen der Reaktionsmischung gegen Standard (Reinsubstanz, GC/MS silyliert) ermittelt

**Tabelle 2:**

| Beispiel | Lösungsmittel | Katalysator 1 (eq) | Katalysator 2 (eq) | Ausbeute d.Th.[%] |
|---|---|---|---|---|
| 39 | 1,2-Dichlorethan | Triphenylphosphanoxid (0,1) | / | 65 |
| 40 | 1,2-Dichlorethan | Triphenylphosphanoxid (0,1) | Aliquat 336 (0,1) | 80 |
| 44 | 1,2-Dichlorethan | Triphenylphosphanoxid (0,1) | Aliquat 175 (0,1) | 77 |
| 45 | 1,2-Dichlorethan | 4-Dimethylaminopyridin 0,1 | / | 81 |
| 46 | 1,2-Dichlorethan | / | Tetrabutylharnstoff (0,1) | 44 |

### Beispiel 47 für die Herstellung von 4,6-Dichlor-5-fluorpyrimidin aus 4,6-Dihydroxy-5-fluorpyrimidin

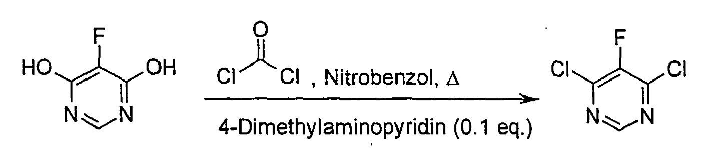

In einem Dreihalskolben mit Rührer, Innenthermometer sowie einem auf -30°C gekühlten Rückflusskühler mit aufgesetzter Gasableitungskappe und Anschluss an einen Waschturm werden 26 g (200 mmol) 4,6-Dihydroxy-5-fluorpyrimidin in 234 g Nitrobenzol vorgelegt und mit 2.4 g 4-Dimethylaminopyridin als Katalysator versetzt. Anschließend werden über 3 Stunden 5 eq. Phosgen bei ca. 85 - 70°C eingeleitet und für 19 Stunden bei 70°C unter Phosgenrückfluss gerührt. Vor der Aufarbeitung wird überschüssiges Phosgen bei abgeschaltetem Kühler weitestgehend abgeleitet (Waschturm). Das Produkt wird durch Destillation unter vermindertem Druck erhalten. Die Ausbeute wird durch Auswaage und Gehaltsbestimmungen des Destillats gegen Standard (Reinsubstanz, GC/MS silyliert) ermittelt und beträgt 91 % d. Th..

## Patentansprüche

1. Verfahren zur Herstellung der Verbindung der Formel 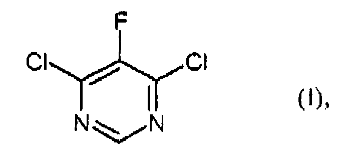 **dadurch gekennzeichnet, dass** man
4,6- Dihydroxy-5-fluor-pyrimidin der Formel (II) oder dessen Alkalisalz 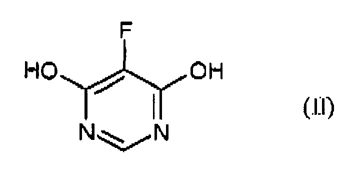 mit Phosgen in Gegenwart von Nitrobenzol als Lösungsmittel und in Gegenwart eines Katalysators umsetzt.

2. Verfahren nach Anspruch 1, **dadurch gekennzeichnet, dass** das Verfahren mit 4-Dimethylaminopyridin als Katalysator durchgeführt wird.

3. Verfahren nach Anspruch 1 oder 2, **dadurch gekennzeichnet, dass** das Verfahren mit einem Trialkylphosphanoxid als Katalysator durchgeführt wird

4. Verfahren nach Anspruch 1 oder 2, **dadurch gekennzeichnet, dass** das Verfahren mit einem Triphenylphosphanoxid als Katalysator durchgeführt wird.

5. Verfahren nach einem der Ansprüche 1 bis 4, **dadurch gekennzeichnet, dass** das Verfahren bei Temperaturen von 40°C bis Rückflusstemperatur der jeweiligen Mischung durchseführt wird.

6. Verfahren nach einem der Ansprüche 1 bis 5, **dadurch gekennzeichnet, dass** zur Herstellung der Verbindung der Formel (I) pro Mol der Verbindung der Formel (11) 2 bis 20 Mol Phosgen eingesetzt werden

7. Verfahren nach einem der Ansprüche 1 bis 6, **dadurch gekennzeichnet, dass** zur Herstellung der Verbindung der Formel (I) pro Mol der Verbindung der Formel (II) 0 bis 30 mol-%, des Katalysators verwendet werden.

## Claims

1. Process for preparing the compound of the formula 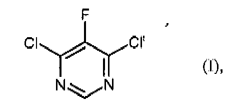 **characterized in that**
4,6-dihydroxy-5-fluoropyrimidine of the formula (II) or its alkali mental salt 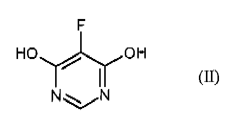 is reacted with phosgene in the presence of nitrobenzene as a solvent and in the presence of a catalyst.

2. Process according to Claim I, **characterized in that** the process is carried out using 4-dimethylaminopyridine as a catalyst.

3. Process according to Claim 1 or 2, **characterized in that** the process is carried out using a trialkylphosphine oxide as a catalyst.

4. Process according to Claim 1 or 2, **characterized in that** the process is carried out using a triphenylphosphine as a catalyst.

5. Process according to any of Claims 1 to 4, **characterized in that** the process is carried out at temperatures from 40°C to the reflux temperature of the particular mixture.

6. Process according to any of Claims 1 to 5, **characterized in that**, to prepare the compound of the formula (I), from 2 to 20 mol of phosgene are used per mole of the compound of the formula (II).

7. Process according to any of Claims 1 to 6, **characterized in that**, to prepare the compound of the formula (I), from 0 to 30 mol% of the catalyst are used per mole of the compound of the formula (II).

## Revendications

1. Procédé pour la préparation du composé de la formule 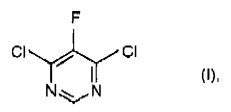 **caractérisé en ce que** l'on fait réagir
la 4,6-dihydroxy-5-fluoro-pyrimidine de la formule (II) ou un sel alcalin de celle-ci 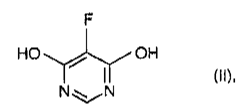 avec du phosgène en présence de nitrobenzène comme solvant et en présence d'un catalyseur.

2. Procédé selon la revendication 1, **caractérisé en ce que** l'on réalise le procédé avec de la 4-diméthylaminopyridine comme catalyseur.

3. Procédé selon la revendication 1 ou 2, **caractérisé en ce que** l'on réalise le procédé avec un phosphanoxyde de trialkyle comme catalyseur.

4. Procédé selon la revendication 1 ou 2, **caractérisé en ce que** l'on réalise le procédé avec un phosphanoxyde de triphényle comme catalyseur.

5. Procédé selon l'une quelconque des revendications 1 à 4, **caractérisé en ce que** l'on réalise le procédé à des températures de 40°C jusqu'à la température de reflux du mélange correspondant.

6. Procédé selon l'une quelconque des revendications 1 à 5, **caractérisé en ce que** l'on utilise pour la préparation du composé de la formule (I) par mole du composé de la formule (II) de 2 à 20 mol de phosgène,

7. Procédé selon l'une quelconque des revendications 1 à 6, **caractérisé en ce que** l'on utilise pour la préparation du composé de la formule (I) par mole du composé de la formule (II) de 0 à 30 % en mole du catalyseur.
